# EUROPEAN PATENT APPLICATION

(11) **EP 4 751 651 A1**
(43) Date of publication of application: **03.06.2026**
(21) Application number: 24216405.1
(22) Date of filing: 29.11.2024
(51) Int. Cl.: A61B 6/04, A61B 6/00

(54) **CAMERA-BASED COUCH MOTION TRACKING FOR IMPROVED MEDICAL IMAGE QUALITY**

(71) Applicant: Koninklijke Philips N.V., 5656 AE Eindhoven (NL)
(72) Inventor: SENEGAS, Julien Thomas, Eindhoven (NL); KOEHLER, Thomas, Eindhoven (NL)
(74) Representative: Philips Intellectual Property & Standards

(57) **Abstract**

A method and a system for acquiring and reconstructing a medical image of a subject supported by a movable couch are provided, as well as an imaging arrangement, and a computer program for carrying out the method. The method for acquiring a medical image of a subject comprises steps of receiving medical image data of the subject, and receiving a sequence of camera images of the subject. The camera images are acquired using one or more optical cameras mounted onto the medical imaging device. The camera images are synchronized with the medical image data. The method also comprises extracting a couch motion from the camera images, which comprises detecting displacement and/or deformation of the couch, and transforming the detected displacement and/or deformation to form the couch motion. The method further comprises performing motion compensated medical image reconstruction of the medical image data using the couch motion.

## Description

### FIELD OF THE INVENTION

The invention generally relates to medical image acquisition. In particular, but not exclusively, the invention relates to computed tomography image acquisition.

### BACKGROUND OF THE INVENTION

In medical imaging, techniques such as computed tomography (CT) and magnetic resonance imaging (MRI) are used to visualize patient anatomy. A CT imaging system generally includes a source of radiation such as x-ray radiation mounted on a rotatable gantry opposite a detector array including one or more rows of detector pixels. The x-ray source rotates around an examination region located between the x-ray tube and the detector array and emits radiation that traverses the examination region and a subject disposed therein. The detector array detects radiation that traverses the examination region and generates projection data indicative of the examination region and the object or subject disposed therein. The projection data is reconstructed to generate volumetric image data indicative of the subject. The volumetric image data can be processed to generate one or more images that include the scanned portion of the subject.

CT imaging is often performed while the subject to be scanner travels through the scanner, either continuously as in helical scans, or step by step, as in axial scans. In both cases, data acquisition and reconstruction assume that the subject moves along a rectilinear trajectory, which is strictly parallel to the axis of rotation of the scanner. In practice, however, the movement of the subject can be different. Vertical couch flexion is a frequent event, which is caused by the subject's weight. This issue can be compensated for by post-processing of the image, or in reconstruction by using a model of the couch trajectory. However, both these approaches lack accuracy.

In addition to vertical couch flexion, other deviations from the ideal trajectory can happen as a result of lateral couch flexion and other displacements and deformations of the couch, which may be caused by the patients' weight, but can also be caused by or mechanical inaccuracies in the design of the subject support. Deviations from the ideal, rectilinear couch trajectory can have consequences on image quality. It can lead to blurring in helical scans and discontinuities in the image slices in axial scans.

### SUMMARY OF THE INVENTION

The current invention seeks to provide an approach for medical image acquisition with improved image quality.

Thereto a method for acquiring a medical image of a subject supported by a movable couch and a system for reconstructing a medical image of a subject supported by a movable couch are provided, as well as an imaging arrangement for acquiring a medical image of a subject and a computer program product comprising instructions for causing a processor to carry out the method for acquiring medical image data of a subject.

The method for acquiring a medical image of a subject supported by a movable comprises steps of receiving medical image data of the subject acquired by a medical imaging device; and receiving a sequence of camera images of the subject, which camera images are acquired using one or more optical cameras mounted onto the medical imaging device. The camera images are synchronized with the medical image data. The method also comprises a step of extracting a couch motion from the sequence camera images, wherein extracting comprises detecting displacement and/or deformation of the couch in the sequence of camera images; and transforming the detected displacement and/or deformation to form the couch motion. The method further comprises performing motion compensated medical image reconstruction of the medical image data using the couch motion. The method is preferably computer-implemented or implemented by other suitable calculation means.

Preferably, wherein the one or more optical cameras are mounted onto at least one of: the stationary gantry of the medical imaging device, and the movable couch of the medical imaging device. These positions allow a good view of the motion of the movable couch relative to the stationary gantry of the imaging device.

Preferably, the one or more optical cameras have a field of view which comprises at least part of a scan volume of the imaging device. More in particular, in case the cameras are mounted onto the stationary gantry of the imaging device, the field of view comprises at least the part of a scan volume that comprises the couch. Including at least part of the scan volume in the field of view allows for extraction of the couch motion in the scan volume, which can improve accuracy of the extracted motion.

In an optional embodiment of the method, the one or more optical cameras are mounted onto the medical imaging device at an angle relative to a vertical plane along a system rotation axis. This configuration allows for easier detection of displacement and/or distortion of the couch in the camera images
In another option that can be used additionally or alternatively, the one or more optical cameras are mounted onto the gantry with an optical axis oriented substantially perpendicular to the system rotation axis. This configuration also allows for easier detection of displacement and/or distortion of the couch in the camera images. In addition, this option makes it possible to use a 2D optical camera. Such a 2D camera can, for example, be used in combination with a prior calibration of the images using a calibration pattern such as a checkerboard pattern.

In a preferred option, the one or more optical cameras are 3D cameras comprising depth sensing. The cameras can be video cameras that are monochrome or color cameras. The spectrum of the cameras may also include at least part of the infrared spectrum.

Additionally or alternatively, multiple cameras are used and the fields of view of the cameras overlap in the scan volume. During acquisition of the medical image data, the subject can obscure part of the field of view of a single camera. This problem can be alleviated by using additional cameras with an overlapping field of view.

In an embodiment of the method, the displacement and/or deformation is detected using one or more pre-defined markers. The pre-defined markers may be part of the movable couch, part of a stationary part of the medical imaging device, such as the stationary gantry, or both. Pre-defined markers can also be placed in the examination room, for example on the walls, ceiling or floor. Preferably, these markers comprise at least one of: one or more edges of the couch, grid lines on the couch surface, grid lines on the stationary gantry, landmarks identified on the couch, landmarks identified on the stationary gantry, markers placed on the couch in pre-defined positions, markers placed on the stationary gantry in pre-defined positions, and markers placed in the examination room at pre-defined positions. Markers that are place int the examination room can be geometric Figures such as squares or circles of a pre-defines size, lines, or images such as grids. Markers have the advantage of being easily identifiable in the camera images. This enables easier and more reliable detection of couch displacement and/or deformation. Having markers on the couch can be sufficient to improve detection of couch displacement and/or deformation when the cameras are mounted onto the stationary gantry. Having markers on the stationary gantry can be sufficient to improve detection of couch displacement and/or deformation when the cameras are mounted onto the movable couch. Quality of detection of displacement and/or deformation of the couch can be further improved by combining markers on both the stationary gantry as reference and the movable couch.

It is particularly advantageous to use one or more edges of the couch as makers. When using the couch edges, no additional markers need to be provided. Moreover, the edge of the couch will not be covered by the subject and is therefore always visible to at least one of the cameras.

In a further embodiment of the method that uses pre-defined markers, detecting displacement and/or deformation of the couch in the sequence of camera images comprises identifying a position of the one or more pre-defined markers in consecutive camera images, and calculating the differences between the positions of the one or more markers between consecutive images. In this embodiment transforming the detected displacement and/or deformation to form the couch motion comprises combining the calculated differences. Combining the couch motion can, for example, be done by parametrizing the couch motion.

According to one aspect of the method, the couch motion comprises an average vertical and/or horizontal motion of the couch in the scan volume. In particular, such an average motion is a spatial average vertical and/or horizontal motion of the couch. In many situations, the couch displacement and/or deformation will not be substantial, in particular in the lateral direction. In these cases, couch motion can be sufficiently accurately described by an average trajectory comprising average horizontal and or vertical motion of the couch. Such an average is computationally efficient for use in motion compensated image reconstruction. In this aspect, couch motion can be expressed as parametrized couch motion in the form of a polynomial or a set of splines.

According to another alternative aspect of the method, the couch motion comprises vertical and/or horizontal motion of the couch at multiple locations in the scan volume. When couch displacement and/or deformation are more pronounced or more complicated, determining horizontal and/or vertical motion at multiple locations improves accuracy. In this aspect, the couch motion can, for example, be expressed as a motion of the couch voxels in the scan volume, or by a deformation vector field.

In the method for acquiring medical image data of a subject, the medical image data that is received can comprise a single set of image data, for example diagnostic image data that is obtained from a diagnostic imaging scan. In practice, more than one image can be acquired of a subject during the image acquisition session. Typically, the subject will remain in the same position on the movable couch during the session. Couch motion will therefore be substantially the same in each medical image data acquisition during the imaging session. In this case, the medical image data can also comprise two or more sets of image data, such as first image data acquired during acquisition of a first medical image and second image data acquired during acquisition of a second medical image of the subject. For example, the first image data can be surview data and the second image data can be diagnostic image data. Alternatively, the first and second image data can both be diagnostic image data, for example in situation where first a non-contrast enhanced scan is acquired followed by a contrast enhanced scan.. In a further alternative, the first and second sets of image data can be acquired by each section of a combined imaging modality such as a PET-CT scanner.

When the medical image data comprises first image data and second image data, the camera images can be synchronized with the first image data to extract a first couch motion, the second image data to extract a second couch motion or both. Motion compensated image reconstruction can also be performed for either the first, or the second image data, or for both. In an exemplary option, the camera images are synchronized with the first image data to extract a first couch motion, and the motion reconstruction is performed on the second image data using the first couch motion. In this option, extraction of the couch motion can start as soon as the first image data is received, and it is not necessary to wait for the second image acquisition to complete. This is a computationally efficient approach. In an alternative option, the camera images can be synchronized with both the first image data and the second image data. The first couch motion that is extracted from the camera images that are synchronized with the first image data can then be used in the extraction of the second couch motion. This can improve the accuracy of the second couch motion extracted from the camera images that are synchronized with the second image data.

The system for reconstructing a medical image of a subject supported by a movable couch comprises an input configured to receive medical image data of the subject acquired by a medical imaging device; an input configured to receive a sequence of camera images of the subject; a couch motion extractor configured to extract a couch motion from the sequence camera images; and an image reconstruction processor configured to perform motion compensated image reconstruction of the medical image data using the couch motion. The camera images are acquired using one or more optical cameras mounted onto the medical imaging device, and the camera images are synchronized with the medical image data. Extracting the couch motion comprises detecting displacement and/or deformation of the couch in the sequence of camera images; and transforming the detected displacement and/or deformation to form the couch motion.

Preferably, the medical image data of the subject is received from one or more of: a computed tomography imaging apparatus, a magnetic resonance imaging apparatus, a positron emission tomography apparatus, an imaging data storage device, and a database such as a dedicated imaging data storage database, hospital database or patient record database.

The imaging arrangement for acquiring a medical image of a subject, comprises an imaging device and the above described system configured to reconstruct a medical image. The imaging device comprises a stationary gantry; a movable couch for supporting the subject; and one or more optical cameras mounted onto the stationary gantry and/or the movable couch. Preferably, the arrangement further comprises a display for displaying the sequence of camera images acquired with the one or more optical cameras. This had the advantage that the subject can additionally be monitored by an operator during the image acquisition procedure.

The computer program product comprising instructions for causing a processor to carry out the above described method, when the computer program is executed.

An advantage of the current invention is that couch displacement and/or deformation can be determined more accurately, in particular in the scan volume. From the couch deformation and/or displacement the motion of the subject due to this deformation and/or displacement can also be determined more accurately. With this, the quality of the motion compensated reconstructed image is also improved.

### BRIEF DESCRIPTION OF THE FIGURES

In the following drawings:
Fig. 1 schematically and exemplarity illustrates an imaging arrangement for acquiring a medical image of a subject comprising a system for reconstructing a medical image.
Fig. 2 schematically illustrates an example of a method for acquiring a medical image of a subject supported by a movable couch.
Figs. 3a and 3b schematically illustrate examples of side views of a medical imaging device comprising cameras mounted to the stationary gantry of the device.
Fig 3c shows a schematic example of a front cross-sectional view of medical imaging device with two cameras mounted to the stationary gantry of the device.
Figs. 4a and 4b schematically illustrate alternative examples of a top view of a medical imaging device comprising one and two cameras mounted to the stationary gantry of the device.
Fig. 5 schematically illustrates a further alternative example as a side view of a medical imaging device comprising a camera mounted to the movable couch of the device.
Figs. 6a and 6b schematically illustrate examples of displacement and/or deformation of the couch.
Fig. 7 schematically illustrates a further example of a method for acquiring a medical image of a subject supported by a movable couch.

### DETAILED DESCRIPTION OF THE INVENTION

In the examples below, a medical image is acquired for a subject. Such a subject can be a human being, particularly a human patient requiring imaging for medical purposes. However, alternative subjects are also envisaged, for example animals such as research animals, pets, or livestock. The approach according to the invention can also be applied to inanimate objects that may be studied using a medical imaging system. In the examples, the imaging device is illustrated as a computed tomography (CT) scanner, but other devices may also be used to acquire the medical image data.

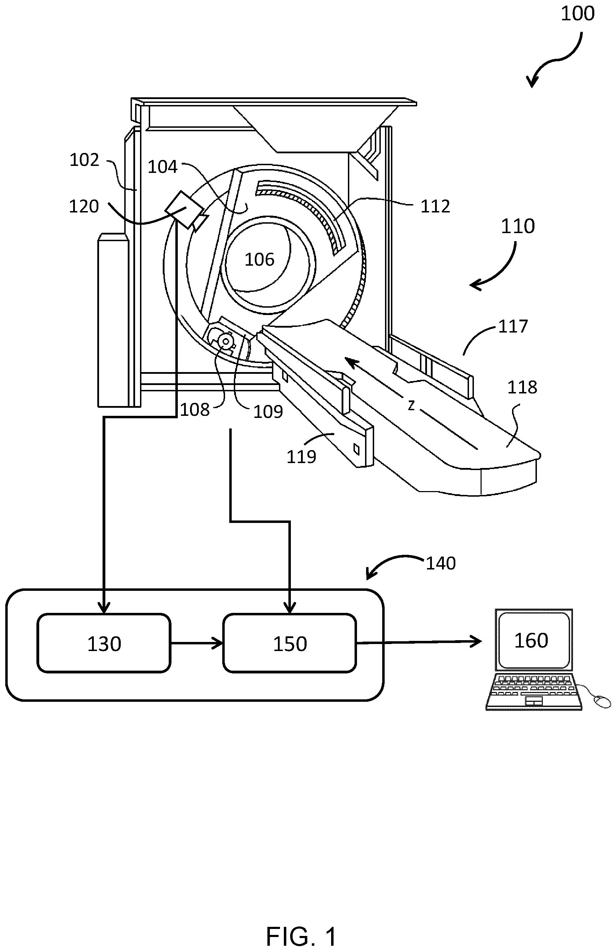

Fig. 1 illustrates an imaging arrangement 100 for acquiring a medical image of a subject.

The imaging arrangement 100 comprises an imaging device 110. The imaging apparatus in this exemplary embodiment is a CT imaging apparatus in the form of a CT scanner. The CT imaging apparatus 110 includes a stationary gantry 102 and a rotating gantry 104, which is rotatably supported by the stationary gantry 102. When the system is in operation, the rotating gantry 104 rotates around an examination region 106 about a longitudinal or z-axis. This axis is also referred to as the system rotation axis. A radiation source 108, such as an x-ray tube, is supported by and rotates with the rotating gantry 104 and emits radiation that traverses the examination region 106. A source collimator 109 collimates the emitted radiation to form a generally fan, wedge, or cone shaped radiation that traverses the examination region 106. The section of the examination region that is traversed by the emitted radiation is referred to as the scan volume.

A table 117 is provided to support a subject to be imaged in the examination region 106. The table comprises a stationary support 119 and a movable couch 118, which is mounted on the support 119. The subject lies on the movable couch 118 for the imaging procedure. Couch 118 is movable along the z-axis in coordination with the rotation of the rotating gantry 104 to facilitate the desired scanning trajectory, for example a stepwise axial trajectory or a helical scanning trajectory.

A radiation sensitive detector array 112 detects radiation emitted by the radiation source 108 that traverses the examination region 106 and generates projection image data indicative of the detected radiation. The illustrated radiation sensitive detector array 112 includes one or more rows of radiation sensitive photosensor pixels along the z-axis.

The imaging arrangement further includes an optical camera 120 mounted the CT imaging apparatus 110. In Fig. 1 the camera is mounted to a stationary part of the CT imaging apparatus, in particular the stationary gantry 102. Alternatively or additionally, a camera can also be mounted onto the movable couch 118. The camera can, for example, be a 3D camera comprising depth sensing. The camera may alternatively be a 2D camera.

In this example, the imaging arrangement 100 further comprises an image reconstruction system 140, which reconstructs the projection data and generates volumetric image data indicative of the examination region 106, including structure of a subject disposed therein. One or more images can be generated from the volumetric image data. The imaging arrangement 100 also comprises an optional display 160, for displaying the one or more generated images.

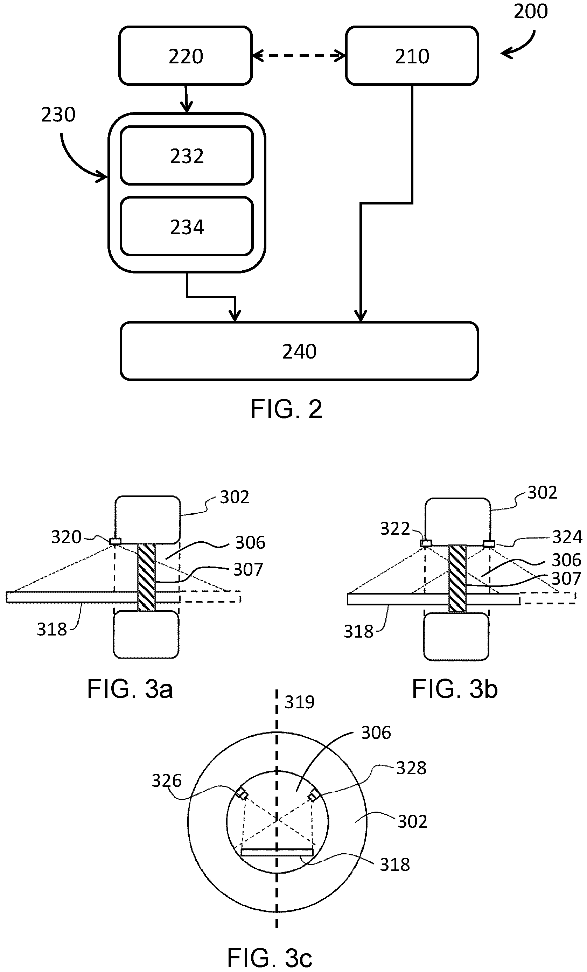

The image reconstruction system 140 is configured to receive medical image data acquired by the CT imaging apparatus 110, and camera images acquired by the optical camera 120. The system comprises a couch motion extractor 130, which calculates the motion of the couch using the camera images. The reconstruction system 140 further comprises a motion compensated image reconstruction unit 150. Image reconstruction system 140 is configured to perform a method for reconstructing a medical image which will be described in further detail with reference to Fig. 2.

Fig. 2 schematically illustrates an example of the method for acquiring a medical image 200 of a subject supported by a movable couch, in particular a CT image of the subject.

The method for acquiring a medical image 200 of the subject supported by the movable couch comprises steps of receiving medical image data of the subject 210 acquired by a medical imaging device; and receiving a sequence of camera images of the subject 220, which camera images are acquired using one or more optical cameras mounted onto the medical imaging device. The camera images are synchronized with the medical image data, as is indicated by the dashed arrow.

The medical image data of the subject can be received directly from an imaging apparatus such as illustrated for CT scanner 110 in Fig. 1. Alternatively, the imaging data can be received from a storage device or a database such as a dedicated imaging data storage database, hospital database or patient record database. The camera image can also be received directly from the one or more cameras mounted on the imaging device, such as camera 120 illustrated in Fig. 1. Alternatively, these images can be stored in and received from a storage device or hospital database. In this case, the camera images are preferably stored together with the medical image data.

The method illustrated in Fig. 2 further comprises a step of extracting a couch motion from the sequence camera images 230, wherein extracting comprises detecting displacement and/or deformation of the couch in the sequence of camera images 232; and transforming the detected displacement and/or deformation to form the couch motion 234.

The method also further comprises performing motion compensated medical image reconstruction 240 of the medical image data using the extracted couch motion.

In the imaging device, there are various options for camera placement. Figs. 3a and 3b show side views of a CT scanner with optical cameras mounted onto a stationary gantry of the medical imaging device.

Fig. 3a shows an example with a single camera 320 located at one side of a gantry 302 having an opening 306 through which the movable couch 318 extends. The opening 306 is also referred to as the bore of the medical imaging scanner. The scan volume inside the bore is indicated with hatched area 307. In this schematic example, the couch is shown moving straight through the bore. This is merely for illustrative purposes, and in practice some deformation such as movement and flexing will take place.

In this example, the camera is mounted by fixing it to the front edge of the stationary gantry with the field of view directed downwards towards the couch. Alternatively, or additionally, a camera can also be mounted more inwards in bore 306 closer to the scan volume 307. Preferably, the camera is mounted with the optical axis substantially perpendicular to the system rotation axis. In this configuration the couch would move in a straight line on the images if no couch deformation would take place. This enables easier detection of displacement and/or distortion in images of a 3D camera. This also makes it possible to use a 2D optical camera. Such a 2D camera can, for example, be used in combination with a prior calibration of the images using a calibration pattern such as a checkerboard pattern.

The camera 320 in Fig. 3a has a field of view which is sufficiently wide in the length direction of the couch to cover the entire length of the couch, and the field of view is sufficiently wide in the width direction of the patient support to cover the whole width of the patient support. Furthermore, the field of view covers the patient support over its entire range of movement. Having a wide field of view has the advantage that more information on couch displacement and/or distortion can be detected in the sequence of images. However, cameras that have a wide field of view, for example fish-eye cameras, tend to have more image distortion towards the edges of the field of view. It is advantageous to use a camera with a field of view that comprises at least part of the scan volume 307 of the imaging device. In Figs 3a and 3b the scan volume 307 is indicated as a hatched area for illustrative purposes. Because the scan volume is the location where the radiation traverses the subject, having at least part of this volume in the field of view allows for more accurate extraction of the couch motion during the medical image acquisition.

Having a wide field of view has the advantage that the optical camera may additionally be used for monitoring the patient. However, having such a large field of view is not mandatory. The camera field of view may instead be narrower to only cover the bore of the medical imaging device, or even just the scan volume of the medical imaging device. An advantage of having a narrower field of view is that the camera images can have less distortions.

Fig. 3b shows a side view of a CT scanner with two cameras; a first camera 322 located at one side of the gantry 302 and a second camera 324 located at an opposite side of the gantry, at different positions along direction of movement of the couch. With this configuration, optical camera images of the couch and the subject can be acquired at two different angles. This can be advantageous to obtain additional images of couch deformation. The subject that is being scanned in the CT scanner can partly block the field of view for one camera. By using a second camera, the additional camera can have a different field of view to acquire images of the subject and the couch that are blocked for the first camera. Alternatively, there may also be more than two cameras. Multiple cameras can be mounted on opposite sides of the gantry, but one or more and also be mounted at the same side of the gantry. Also, multiple cameras can be mounted at various different positions, along the patient support.

Preferably, the fields of view of the one or more cameras overlap in the scanner bore 306, and even more preferably in the scan volume 307 of the scanner. This is illustrated in Fig. 3b, where part of the scan volume 307 is comprised in the field of view of both cameras 322 and 324.

Fig. 3c shows a schematic example of a front cross-sectional view of medical imaging device in the form of a CT scanner with two cameras mounted to the stationary gantry at an angle with respect to a volume perpendicular to the couch. The CT imaging apparatus has a circular bore 306 at its center. Two optical cameras 326 and 328 are shown, which may be located within the gantry bore 306, at either side of the gantry 302, or at one side of the gantry 302. The use of two cameras is merely an example, one or more could also be used. The cameras 326 and 328 are each mounted onto the gantry 302 at an angle relative to the vertical plane 319 along to the system rotation axis. As a result, the cameras face the patient support in the scan volume from a lateral side rather than from directly above. This ensures that both horizontal and vertical motion is evident in the images. The cameras may be mounted at the same angle or at different angles. In the example shown in Fig. 3c, the cameras are mounted at an angle of about 45 degrees relative to plane 319 perpendicular to the couch. This results in a relative angle of about 90 degrees between the cameras.

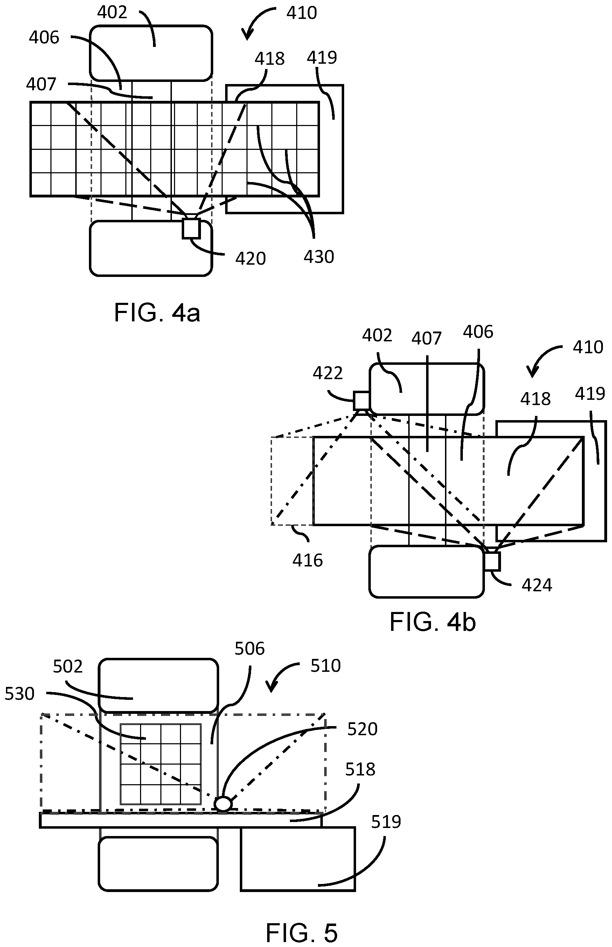

Fig. 4a schematically illustrates an example of a top view of a medical imaging device 410 comprising a camera 420 mounted to the stationary gantry 402 of the device. The imaging device 410 further comprises a movable couch 418 and a stationary couch support 419. The stationary couch support supports movable couch 418 and enables this couch to move into and out of examination region 406 within the gantry of the imaging device. The scan volume 407 is a smaller region within the examination region 406.

The optical camera 420 is mounted inside the examination region, and next to, but not inside the scan volume. The optical axis of camera 420 is substantially perpendicular to the rotation axis of the imaging device. The field of view of camera 420 is indicated with dashed lines. In this example the field of view comprises the volume of the examination region 406 that comprises the couch, which includes the part of the scan volume 407 that comprises the couch. The field of view of camera 420 extends somewhat beyond the examination region, but does not cover the entire surface and range of the couch 418.

In the example of Fig. 4a, couch 418 is provided with pre-defined markers in the form of grid lines 430. The grid lines shown are schematic and may implemented as a courser or finer grid according to the accuracy needed. The grid lines enable easier and more reliable detection of couch displacement and/or deformation. In the camera images couch deformation and displacement will show as deformation of the grid when observed over consecutive frames. Having grid lines covering substantially the entire couch ensures that at least part of the grid lines will remain visible when the subject is lying on the couch during image acquisition. Alternative pre-defined markers for the grid lines could also be used. For example, small markers can be placed on the couch in pre-defined positions.

Fig. 4b schematically illustrates an alternative example of a top view of a medical imaging device 410 comprising two cameras 422 and 424 mounted to the stationary gantry 402 of the device. The imaging device 410 further comprises a movable couch 418 and a stationary couch support 419. The stationary couch support 419 supports movable couch 418 and enables this couch to move into and out of examination region 406 within the gantry of the imaging device. The scan volume 407 is a smaller region within the examination region 406.

The two optical cameras 422 and 424 are mounted onto the back and front of the stationary gantry 402 next to the examination region 406. The optical axes of cameras 422 and 424 are both substantially perpendicular to the rotation axis of the imaging device. The fields of view of cameras 422 and 424 are indicated with dash-dot and large dash lines, respectively. In this example the field of view comprises the volume of the examination region 406 that comprises the couch, which includes the part of the scan volume 407 that comprises the couch. The fields of view of the cameras extends beyond the examination region on the side on which the camera is mounted. For each camera, the field of view covers the area up to which the couch extends. For camera 422, which is mounted at the back of the gantry, the field of view include the additional region 416 into which the couch 418 can maximally extend. Camera 424 includes the region of the stationary support 419 into which the couch can maximally retract. In combination, both cameras cover the entire surface and range of the couch 418. Having such a wide coverage, enables the camera images to also be used to view the patient during the imaging procedure. This allows the operator to monitor the patients wellbeing.

In the example of Fig. 4b, the straight edges of couch 418 are used as pre-defined markers. In this example also the fields of view of both cameras 422 and 424 overlap in the examination region 406, including an overlap in the scan volume 407. It can happen that the subject on the table obscures the further edge of the couch from the camera. In this configuration, the edge that is obscured for one camera, will remain visible for the other. In the scan volume 407, both couch edges can always be identified. This allows the couch motion to be determined with an improved accuracy.

Fig. 5 schematically illustrates a further alternative example. This Figure shows a side view of a medical imaging device 510 comprising a camera 520 mounted to the movable couch 518 of the device. This example shows one camera, but multiple cameras can also be mounted to the movable couch 518. The imaging device further comprises a stationary couch support and a stationary gantry 502. The stationary gantry 502 surrounds the examination regions 506. The scan volume is not indicated in this Figure, but lies within the examination region 506.

The camera 520 is mounted onto the movable couch 518. In this example, the camera 520 with the optical axis substantially perpendicular to the rotation axis of the imaging device. However, such a perpendicular orientation is not necessary and the camera may also be angled differently. When the camera is mounted more towards one of the front or back of the couch, it may be advantageous to angle the optical axis towards the stationary gantry 502. In Fig. 5 the field of view is indicated with dash-dot lines and comprises the length of the couch. During image acquisition the field of view of the camera comprises at least part of the stationary gantry, and in particular at least part of the stationary gantry that encircles the examination region. In Fig. 5, the camera 520 has a field of view that is directed to the inner side of the gantry 502. The gantry has pre-defined markers in the form of grid lines 530. Also in this example, the grid lines 530 shown are schematic and may implemented as a courser or finer grid according to the accuracy needed. Alternative pre-defined markers, such as landmarks identified on the gantry, can also be used. Having pre-defined stationary markers allows to determine couch displacement and/or deformation from the camera images as the camera moves with the couch. The ideal trajectory of rectilinear, horizontal motion can be compensated for in extracting the couch motion.

The field of view of the camera 520 can also be adjusted or enlarged to include the upper inside of the stationary gantry and even part of the opposite area inside the examination region 506. The grid lines 530 can also be extended to cover the semi-circular upper half of the inside of the bore of the gantry, or even the entire stationary gantry 520 surface inside examination region 506. Such an extended field of view ensures that part of the markers are always visible and not obstructed by the subject.

I can also be advantageous to combine the examples illustrated in Figs. 4 and 5 to have camera on both the stationary gantry and the movable support. Combining the information can improve the accuracy of the extracted couch motion.

In the examples of Figs. 4 and 5 pre-defined markers are used to detect displacement and/or deformation of the couch in the sequence of camera images. To detect the displacement and/or deformation, the position of the pre-defined markers in consecutive camera images are identified and the differences between the positions of the markers between consecutive images are calculated. The detected displacement and/or deformation are transformed to form the couch motion by combining the calculated differences. Combining the calculated differences can, for example, be done by creating a series of calculated differences, by defining shifts for each couch voxel, or parametrizing the differences.

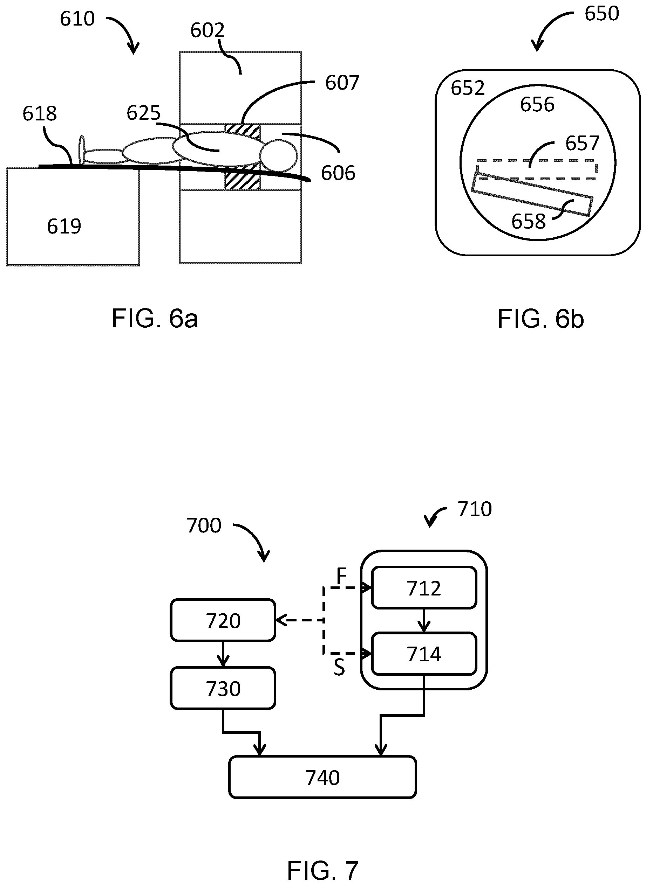

Figs 6a and 6b illustrate examples of displacement and deformation of the couch. The Figures are meant as illustrations and couch deformations and displacements in particular are exaggerated. The deformation and/or displacement is likely to be much less prominent in reality.

Fig. 6a illustrates a couch displacement in the form of vertical flexing. Depending on the degree of flexing, the couch can also display some deformation. Fig. 6a schematically depicts an imaging device 610 in the form of a CT scanner. The CT scanner comprises a stationary gantry 602 with an examination region 606. Within the examination region is scan volume 607, indicated in the Figure as a hatched area. Moveable couch 618 is supported by stationary support 619. Subject 618 lies on the couch and is moved through the scan volume 607 in the bore 606. As the subject 618 is moved in and out of the bore, the couch moves downwards and upwards in the scan volume. As a result, the subject 618 also displaces vertically in the scan volume. Vertical couch flexion as illustrated in Fig. 6a is a frequent event, which is caused by the subject's weight. The amount of flexing is not just determined by the total weight of the subject, but also by their weight distribution and position on the couch. Subject motion, for subjects of the same weight or even for the same subject, can therefore vary for each scan. Determining couch motion during the scan by synchronizing the camera images with the medical image data more can accurately provide the couch motion. Thereby image quality is improved.

In the example of Fig. 6a, there is only substantial motion in the vertical direction. In this case, the couch motion can be sufficiently accurately described by an average vertical motion of the couch. The average vertical motion can, for example, be a vertical trajectory. Such an average is computationally efficient for use in motion compensated image reconstruction. In option, the couch motion can be mathematically conveniently expressed as parametrized couch motion in the form of a fitted polynomial function or a combined fitted set of splines.

In addition to vertical couch flexion, also other deviations from the ideal trajectory can happen. Lateral couch flexion and other displacements can also occur. This may be caused by the patients weight, but can also be caused by mechanical inaccuracies in the design of the subject support. For example, when the direction of movement of the couch is not perfectly aligned with the system rotation axis, this will result in a lateral displacement of the couch in the scan volume. Like vertical flexing, also horizontal displacement and flexing can cause distortion of the couch.

Fig. 6b illustrates a couch displacement in the form of combined vertical and lateral flexing. Fig. 6b schematically depicts an imaging device 650 comprising a stationary gantry 652 with an examination region 656 in the form of a circular bore. Imaging devices with such a configuration are, for example, and CT and MR scanners and PET imaging devices. The ideal trajectory with no couch deformation is indicated by dashed cross-section 657. The location of the displaced couch is indicated by cross-section 658. The couch has flexed vertically resulting in a downwards displacement in the bore 656. Because of this, the subject is also displaced downwards in the scan volume during image acquisition. The couch has also flexed laterally: the right edge of the couch has moved downwards more than the left edge. This horizontal flexing causes a lateral displacement combined with a rotation of the subject in the scan volume. By combining all these forms of motion, the movement of the subject can be more accurately compensated for in the motion compensated reconstruction.

In the example of Fig. 6b, the couch motion comprises vertical and horizontal motion of the couch that is different at multiple locations in the scan volume. In this case, determining horizontal and vertical motion at multiple locations improves accuracy. The couch motion can, for example, be expressed as a motion of the couch voxels in the scan volume, or by a deformation vector field.

Fig. 7 schematically illustrates a further example of a method for acquiring a medical image of a subject supported by a movable couch. The method comprises steps of receiving medical image data 710, receiving synchronized camera images 720, extracting couch motion 730, and performing motion compensated medical image reconstruction of the medical image data using the extracted couch motion.

In this example, the medical image data that is received comprises first image data 712 acquired during acquisition of a first medical image and second image data 714 acquired during acquisition of a second medical image of the subject.

In particular in CT image acquisition, but also in MR image acquisition, an initial surview scan is acquired for planning the diagnostic scan. Such a scan is usually acquired at a low dose of radiation and has a longer scan range than the diagnostic scan. The surview scan can be a 2D image, or a low-dose 3D scan, which can be reconstructed into a 3D surview image. This surview scan can provide first image data. The subsequent diagnostic scan then follows providing second image data. In another option, for PET-CT image acquisition, a CT image is acquired separately first, providing the first image data, before the PET image is acquired providing the second image data.

In cases where more than one diagnostic scan is acquired after scan planning, first, second, or even third image data can be provided by the multiple diagnostic scans. For example, in contrast-enhanced CT imaging, commonly first a non-contrast image is acquired of the subject, followed by contrast injection and acquisition of the contrast-enhanced medical image of the subject. In an alternative situation, it may happen that an immediate rescan of the patient is needed. The initial diagnostic scan may contain artifacts that make it unsuitable for diagnostic purposes. Such a scan can, however, still have sufficient quality information for it to be used as first medical image data for extracting the couch motion.

In a simple embodiment, the first image data that is acquired, such as the surview scan, is only used for planning the second image acquisition, such as the diagnostic scan only. In that option, the first image data may not be part of the medical image data that is received. In this case the camera images 720 are synchronized with the second, e.g. diagnostic, medical image data 714. Optionally, the first image data 712 can be part of the received medical image data, but is not used for extracting the couch motion 730. In this example, the camera images 720 are synchronized with the second, e.g. diagnostic, medical image data 714 only. This is indicated by dashed arrow S in Fig. 7.

Synchronizing the camera image with the second scan, which can be for example the diagnostic image or the contrast-enhanced image, will provide the actual motion of the couch observed during the image acquisition. This can provide a high accuracy for motion compensation of the image used for diagnostic purposes. However, synchronizing the camera images with the acquisition of the second scan can also result in additional complexity and latency of the image reconstruction. In an alternative option, the camera images 720 can be synchronized with the first image data 712. This is indicated in Fig. 7 with dashed arrow F. The couch motion can be extracted efficiently from this scan and used for the motion compensated medical image reconstruction of the diagnostic data. This has the added advantage that it is not necessary to wait for the image acquisition to be completed before the couch motion can be extracted. Because the surview scan typically has a larger scan range, this option can also have the further advantage that the couch motion can be extracted for a wider range. This can improve the accuracy of the motion compensated image reconstruction.

In a further alternative example of a method for acquiring a medical image of a subject supported by a movable couch, the sequence of camera images 720 can be synchronized with both the first image data 712 and the second image data 714, indicated by both dashed arrows F and S combined in Fig. 7. An initial couch motion can be extracted from the camera images synchronized F with the first image data 712. This initial couch motion can then be used as input to improve the extraction of the couch motion from the camera images synchronized S with the second image data 714. Particularly for complex couch deformations this will further improve the accuracy of the couch motion, and the motion compensated image reconstruction.

Additionally or alternatively, the accuracy of the motion compensation can be improved when the first image data is 3D image data. For example, the first image data can be 3D surview image data, an initial screening image acquired at a lower resolution, an initial non-contrast CT scan for a contrast-enhanced image acquisition, an initial CT scan that immediately required rescanning, or a CT image acquired as part of a combined PET-CT scan.

In addition to extracting the couch motion from the camera images, the first image data can also be used to determine displacement and/or deformation of the couch. By combining the information on displacement and/or deformation from the camera images and the first image data, the accuracy of extracted couch motion can be improved. There can be several ways this may be implemented. For example, the couch motion can be extracted separately from the 3D first image data and then be combined with the motion extracted from the camera images. For an ideal couch trajectory, the couch will present as a straight line or bar in the reconstructed CT image. Any deviations from this are due to couch displacement and/or displacement of the couch and can be determined from the 3D image. This can improve accuracy in particular for couch deformations that may be harder to detect from the camera images.

In an alternative example, a motion compensated image reconstruction can be performed on the first image data using the couch motion extracted from the camera images that are synchronized with this first image data. With higher accuracy of the extracted couch motion, the couch will be sharper in the reconstructed medical image. This sharpness criterium can be used as a penalty term in a numerical optimization framework. Through this optimization, the accuracy of the couch motion can be improved.

With a 3D initial image, in addition to the couch motion, any accompanying movement of internal anatomy of the subject can also be analyzed. Any changes in subject anatomy related to couch deformation that is additional to the determined couch motion can be added and used as input for the motion compensated reconstruction of the image of the subject.

Any of the method steps disclosed herein, may be recorded in the form of a computer program comprising instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray^{™} and DVD. Examples of a propagation medium are the Internet or other wired or wireless telecommunication systems.

Variations to the disclosed embodiments can be understood and effected by those skilled in the art in practicing the claimed invention, from a study of the drawings, the disclosure, and the appended claims. It is noted that the various embodiments may be combined to achieve further advantageous effects.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A method for acquiring a medical image (200) of a subject supported by a movable couch, the method comprising the steps of:
- receiving medical image data of the subject (210) acquired by a medical imaging device;
- receiving a sequence of camera images of the subject (220), which camera images are acquired using one or more optical cameras mounted onto the medical imaging device; wherein the camera images are synchronized with the medical image data;
- extracting a couch motion from the sequence camera images (230), wherein extracting comprises:
- detecting displacement and/or deformation of the couch in the sequence of camera images (232);
- transforming the detected displacement and/or deformation to form the couch motion (234); and
the method further comprising:
- performing motion compensated medical image reconstruction (240) of the medical image data using the couch motion.

2. The method according to claim 1, wherein the one or more optical cameras are mounted onto at least one of: the stationary gantry of the medical imaging device, and the movable couch of the medical imaging device.

3. The method according to claim 1 or 2, wherein the one or more optical cameras have a field of view which comprises at least part of a scan volume of the imaging device.

4. The method according to any of claims 1-3, wherein the one or more optical cameras are mounted onto the medical imaging device with an optical axis oriented substantially perpendicular to the system rotation axis.

5. The method according to any of claims 1-4, wherein the one or more cameras comprise multiple cameras and the fields of view of the cameras overlap in the scan volume.

6. The method according to any of claims 1-5, wherein the displacement and/or deformation is detected using one or more pre-defined markers

7. The method according to claim 6, wherein;
the markers comprise at least one of: one or more edges of the couch, grid lines on the couch surface, grid lines on the stationary gantry, landmarks identified on the couch, landmarks identified on the stationary gantry, markers placed on the couch in pre-defined positions, markers placed on the stationary gantry in pre-defined positions, and markers placed in the examination room at pre-defined positions.

8. The method according to claim 6 or 7, wherein detecting displacement and/or deformation of the couch in the sequence of camera images comprises:
- identifying a position of the one or more pre-defined markers in consecutive camera images; and
- calculating the differences between the positions of the one or more markers between consecutive images; and
wherein transforming the detected displacement and/or deformation to form the couch motion comprises combining the calculated differences.

9. The method according to any of claims 1-8, wherein the couch motion comprises an average vertical and/or horizontal motion of the couch in the scan volume.

10. The method according to any of claims 1-8, wherein the couch motion comprises vertical and/or horizontal motion of the couch at one or more locations in the scan volume.

11. The method according to any of claims 1-10, wherein the medical image data comprises first image data acquired during acquisition of a first medical image of the subject and second image data acquired during acquisition of a second medical image of the subject; wherein the camera images are synchronized with the first image data to extract a first couch motion; and wherein the motion compensated image reconstruction is performed on the second image data using the first couch motion.

12. The method according to any of claims 1-10, wherein the medical image data comprises first image data acquired during acquisition of a first medical image of the subject and second image data acquired during acquisition of a second medical image of the subject; wherein the camera images are synchronized with the first image data to extract a first couch motion; wherein the camera images a further synchronized with the second image data to extract a second couch motion using the first couch motion; and wherein the motion compensated image reconstruction is performed on the second image data using the second couch motion.

13. A system for reconstructing a medical image (140) of a subject supported by a movable couch, comprising:
- an input configured to receive medical image data of the subject acquired by a medical imaging device (110);
- an input configured to receive a sequence of camera images of the subject, which camera images are acquired using one or more optical cameras (120) mounted onto the medical imaging device (110); wherein the camera images are synchronized with the medical image data;
- a couch motion extractor (130) configured to extract a couch motion from the sequence camera images, wherein extracting the couch motion comprises:
- detecting displacement and/or deformation of the couch in the sequence of camera images;
- transforming the detected displacement and/or deformation to form the couch motion; and
the system further comprising:
- an image reconstruction processor (150), which is configured to perform motion compensated image reconstruction of the medical image data using the couch motion.

14. An imaging arrangement (100) for acquiring a medical image of a subject, comprising:
- an imaging device (110) comprising:
- a stationary gantry (102);
- a movable couch (118) for supporting the subject;
- one or more optical cameras (120) mounted onto the stationary gantry (102) and/or the movable couch (118);
- the system for reconstructing a medical image according to claim 13.

15. A computer program product comprising instructions for causing a processor to carry out the method according to any of claims 1-12, when the computer program is executed.
